# EUROPEAN PATENT APPLICATION

(11) **EP 4 327 740 A1**
(43) Date of publication of application: **28.02.2024**
(21) Application number: 21917916.5
(22) Date of filing: 16.12.2021
(51) Int. Cl.: A61B 5/145, A61B 5/1477, A61B 5/155, A61B 5/00, G01N 27/327

(54) **MICRONEEDLE BIOSENSOR AND MANUFACTURING METHOD FOR SAME**

(30) Priority: 22.04.2021 KR 20210052167; 14.09.2021 KR 20210122493
(71) Applicant: Albiti Inc., Seoul 06035 (KR)
(72) Inventor: AHN, Jun Young, Palo Alto, California 94306 (US); JANG, Eun Hee, Seoul 04733 (KR)
(74) Representative: ABG Intellectual Property Law, S.L.
(86) International application number: PCT/KR2021/019230
(87) International publication number: WO 2022/149754

(57) **Abstract**

The present invention provides a microneedle biosensor including a working electrode including a first base of a circular thin film type, a plurality of microneedles which perpendicularly protrudes on the first base, and a first wiring line which extends from one end of a circumference of the first base; a counter electrode which includes a second base of a strip thin film type having a 3/4 circular shape which is spaced apart from a circumference of the first base with a setting distance to be concentric with the first base, a plurality of microneedles which perpendicularly protrudes on the second base, and a second wiring line which extends from one end of the second base to be horizontally disposed with the first wiring line; and a reference electrode which includes a third base of a strip thin film type having a 1/4 circular shape which is spaced apart from the other end of the second base with a setting distance and is spaced apart from the circumference of the first base with a setting distance to be concentric with the first base, a plurality of microneedles which perpendicularly protrudes on the third base, and a third wiring line which extends from one end of the third base, and a manufacturing method thereof.

## Description

### [Technical Field]

The present invention relates to a microneedle biosensor and a manufacturing method thereof.

### [Background Art]

A typical human blood sugar level is in the range of 70 to 130 mg/dL before a meal and in the range of 180 mg/dL after a meal. Cases exceeding this range are classified as hyperglycemia and cases below the normal range are classified as hypoglycemia. Hyperglycemia has a significant correlation with diabetes. Diabetes is a metabolic syndrome in which high blood sugar levels persist for a long period of time.

In order to diagnose diabetes and manage the diabetes so as not to develop into complications, systematic blood sugar measurement and treatment need to be carried out together. According to the typical diabetes management, an amount of insulin to be injected is determined according to a patient's blood sugar level and the insulin is administrated at predetermined time intervals. However, the patient's blood sugar level and the change in blood sugar level in accordance with insulin administration are different for individual patients so that it is difficult to accurately and efficiently determine an amount of insulin to be injected, an injection timing, and interval.

In order to solve this problem, continuous glucose monitoring (CGM) system may be used. The continuous glucose monitoring system was first developed by Medtronic (Minneapolis, MN, USA), approved by U.S. FDA in June 1996 and helps treat diabetic patients who have large blood sugar fluctuations and frequent hypoglycemia. The continuous glucose monitoring system is configured by three parts: a blood sugar sensor, a wireless transmitter, and a receiver. The sensor is inserted into the subcutaneous fat to measure a sugar from the interstitial fluid.

The continuous glucose monitoring system of the related art includes a sensor which is inserted into a body to measure a blood sugar from the blood, a needle which guides the sensor to be inserted into the body, and a separate applicator coupling structure to apply the sensor module to the body. The sensor is placed in a hollow of a syringe needle, subcutaneously pierced by the syringe needle to be inserted into the subcutaneous fat. The sensor is placed in the hollow of the syringe needle. A size of the syringe needle which is used to detect the blood sugar is at most 21 gauge. A sensing strip needs to be disposed in the hollow of the syringe needle so that diameters of the syringe needles used for the continuous glucose measuring system are 600 nm to 800 nm. However, when the diameter of the sensor needle is 600 nm to 800 nm, it causes the pain to the user, which causes the discomfort during the continuous usage.

### [Disclosure]

### [Technical Problem]

The present invention has been made an effort to solve the problem of the related and provide a microneedle biosensor which reduces the pain of the patient during wearing with a minimally invasive manner.

An object of the present invention is to provide a thin film type microneedle biosensor which improves the edge lifting after being attached.

### [Technical Solution]

The present invention provides a microneedle biosensor including a working electrode including a circular thin film first base, a plurality of microneedles which perpendicularly protrudes on the first base, and a first wiring line which extends from one end of a circumference of the first base; a counter electrode which includes a strip thin film second base which forms a part of a second circumference spaced apart from a circumference of the first base with a setting distance to be concentric with the first base, a plurality of microneedles which perpendicularly protrudes on the second base, and a second wiring line which extends from one end of the second base to be horizontally disposed with the first wiring line; and a reference electrode which includes a strip thin film third base which is spaced apart from the other end of the second base with a setting interval and forms a second circumference with the strip shape of the second base, a plurality of microneedles which perpendicularly protrudes on the third base, and a third wiring line which extends from one end of the third base.

The second base forms 3/4 of the circumference and the third base forms 1/4 of the circumference.

Bottom surfaces of the first base, the second base, and the third base are attached onto an adhesive sheet.

The first wiring line perpendicularly extends from one end of the circumference of the first base, the second wiring line extends from one end of the second base to be horizontally disposed with the first wiring line, and the third wiring line extends from one end of the third base to be horizontally disposed with the first wiring line.

Another exemplary embodiment of the present invention provides a manufacturing method of a microneedle biosensor including a) forming a mold by forming a groove corresponding to a shape of a microneedle of each of a working electrode, a counter electrode, and a reference electrode in a solid resin block; b) imprinting a polymer layer of each of the working electrode, the counter electrode, and the reference electrode on the mold using polymer; c) forming a metal layer by forming shadow masks corresponding to patterns of the working electrode, the counter electrode, and the reference electrode and sputtering Au or Au+Ti/Cr adhesive layer; and d) forming a passivation layer on the metal layer.

The manufacturing method further includes, after performing the step d), plating with Pt-black and coating with nafion on tips of the microneedles of the working electrode.

The manufacturing method further includes, after performing the step d), coating the microneedles of the reference electrode with Ag/AgCl.

The solid resin block is a polydimethylsiloxane (PDMS) or polytetrafluoroethylene (PTFE) material.

The polymer in the step b) is an acrylic or PLA material.

In the step c), in the patterns of the working electrode, the counter electrode, and the reference electrode, the working electrode includes a first base of a circular thin film type, the counter electrode includes a second base of a strip thin film type which forms a part of a second circumference spaced apart from the circumference of the first base with a setting distance to be concentric with the first base, and the reference electrode includes a third base of a strip thin film type which is spaced apart from the other end of the second base with a setting interval and forms the second circumference with the strip shaped second base.

### [Advantageous Effects]

According to the exemplary embodiment of the present invention, it is possible to provide a microneedle biosensor which reduces the pain of the user during the wearing in a minimally invasive manner.

According to the exemplary embodiment of the present invention, it is possible to provide a thin film type microneedle biosensor which improves the edge lifting after being attached.

According to the exemplary embodiment of the present invention configured as described above, a microneedle biosensor which accurately senses and is the most suitable for a skin surface shape while reducing the pain of the user during the wearing and a manufacturing method thereof.

### [Description of Drawings]

FIG. 1 is a view illustrating a microneedle biosensor according to an exemplary embodiment of the present invention.
FIG. 2 is a flowchart illustrating a manufacturing process of a microneedle biosensor.
FIG. 3 is a flowchart illustrating a thermal imprinting process, in a PLA microneedle biosensor manufacturing process.
FIG. 4 is a flowchart illustrating a UV imprinting process, in an acrylic microneedle biosensor manufacturing process.
FIG. 5 is a view illustrating a result obtained by performing the step S12 of FIG. 2.
FIGS. 6 to 8 are views illustrating a result obtained by performing the step S13 of FIG. 2.
FIGS. 9 and 10 are views illustrating a needle plated with Pt-black.
FIG. 11 is a view illustrating Pt-black coated with nafion.
FIG. 12 is a view illustrating a coated state with Ag/AgCl and nafion.
FIGS. 13 and 14 illustrate graphs obtained by measuring glucose using a microneedle biosensor according to an exemplary embodiment of the present invention.

### [Best Mode]

The present invention will be described in detail below with reference to the accompanying drawings. Herein, the like configuration is denoted by the like reference numeral and repeated description and the detailed description of a known function and configuration that may make the purpose of the present invention unnecessarily ambiguous will be omitted. The Examples of the present invention are provided for more completely explaining the present invention to those skilled in the art. Accordingly, the shape, the size, etc., of elements in the figures may be exaggerated for explicit comprehension.

The microneedle biosensor according to the exemplary embodiment of the present invention is a minimally invasive microneedle biosensor. The present invention relates to a biosensor which infiltrates the skin with the microneedles to be in contact with the body fluids to monitor biological signals. The biosensor according to the exemplary embodiment of the present invention refers to a sensor which is mounted on the skin surface to continuously measure a blood sugar level during a set period to measure a blood sugar level from interstitial fluid (ISF) of the infiltrated host, but is not limited thereto.

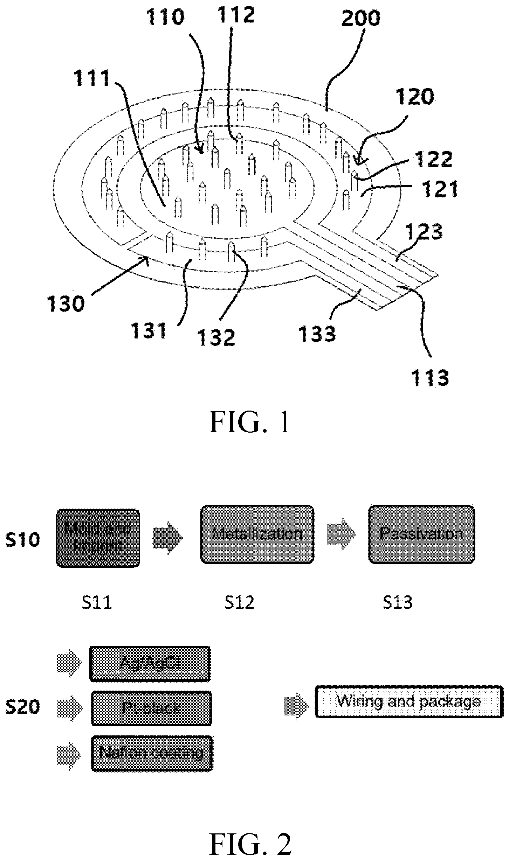

FIG. 1 is a view illustrating a microneedle biosensor according to an exemplary embodiment of the present invention. As illustrated in the drawing, the microneedle biosensor includes a working electrode (WE) 110, a counter electrode (CE) 120, a reference electrode (RE) 130, and an adhesive sheet 200. The working electrode 110 includes a first circular base 111, a plurality of microneedles 112 which perpendicularly protrudes on the first base 111, and a first wiring line 113 which perpendicularly extends from one end of the first base 111. The counter electrode 120 includes a second base 121 which is formed as a 3/4 circular strip with a setting width spaced apart from a circumference of the first base 111 with a setting interval to be concentric with the first base 111, a plurality of microneedles 122 which perpendicularly protrudes on the second base 121, and a second wiring line 123 which extends from one end of the second base 121 to be horizontal with the first wiring line 113. The reference electrode 130 includes a third base 131 which is spaced apart from the other end of the second base 121 with a setting interval and is formed as a 1/4 circular strip with a setting width spaced apart from the circumference of the first base 111 with a setting interval to be concentric with the first base 111, a plurality of microneedles 132 which perpendicularly protrudes on the third base 131, and a third wiring line 133 which perpendicularly extends from one end of the third base 131 to be horizontally disposed with the first wiring line 113.

The counter electrode 120 and the reference electrode 130 are spaced apart from the working electrode 110 with a setting interval to enclose the working electrode 110. The working electrode 110, the counter electrode 120, and the reference electrode 130 are attached onto the adhesive sheet 200. The adhesive sheet 200 is desirably formed by applying an adhesive on one surface of a fiber or polymer sheet. The adhesive sheet 200 desirably has an elasticity in the sheet itself. The working electrode 110, the counter electrode 220, and the reference electrode 130 are attached on a surface of the adhesive sheet 200 applied with an adhesive which is attachable to the skin. The circular working electrode 110 and the counter electrode 120 and the reference electrode 130 which are spaced apart from the working electrode 110 to have a strip shape and enclose the working electrode 110 are disposed and are attached to the fiber or polymer sheet. Accordingly, the working electrode 110, the counter electrode 220, and the reference electrode 130 ensure a sufficient effective area for sensing. Further, when the working electrode 110, the counter electrode 220, and the reference electrode 130 are attached to the human skin which may not be flat due to its structure, the working electrode 110, the counter electrode 220, and the reference electrode 130 are flexibly inclined according to the angle of the skin to be closely attached onto a skin contact surface. That is, when a sensor with a flat base is attached onto the skin which is not flat, after time passes from the attachment, the edges are lifted due to the resilience, but the microneedle biosensor according to the exemplary embodiment of the present invention may solve this problem.

The microneedle biosensor according to the exemplary embodiment of the present invention is formed by sequentially laminating a polymer layer, a metal electrode layer, and a passivation layer.

FIG. 2 is a flowchart illustrating a manufacturing process of a microneedle biosensor. As illustrated in the drawing, the manufacturing method of a microneedle biosensor includes a microneedle manufacturing process S10 configured by a mold and imprint process S11, a metallization process S 12, a passivation process S 13 and a post-processing process S20 configured by Ag/AgCl, Pt-black, and nafion coating and wiring and packaging processes.

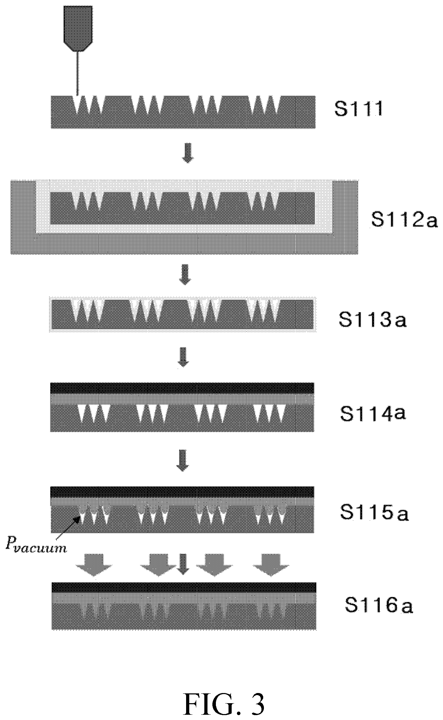

FIG. 3 is a flowchart illustrating a mold and imprint process S11 for forming a PLA polymer layer in the manufacturing process of a microneedle biosensor of FIG. 2.

As illustrated in the drawing, the process is configured by a mold manufacturing step S 111 of forming a groove having a shape corresponding to a microneedle on a polytetrafluoroethylene (PTFE) block with laser, a release agent coating step S 112a of coating a release agent on the mold, a step S 113 a of drying the release agent, a step S114a of forming the PLA layer on the mold with the groove and pressurizing with ceramic, a step S 115a of baking in a vacuum oven at 200°C, and a step S 116a of pressurizing with a press after vacuum off. A PLA (Poly Lactic Acid) microneedle, which is eco-friendly, non-toxic, biodegradable, and biocompatible, is formed. The PLA needle has a high elastic modulus and buckling stiffness.

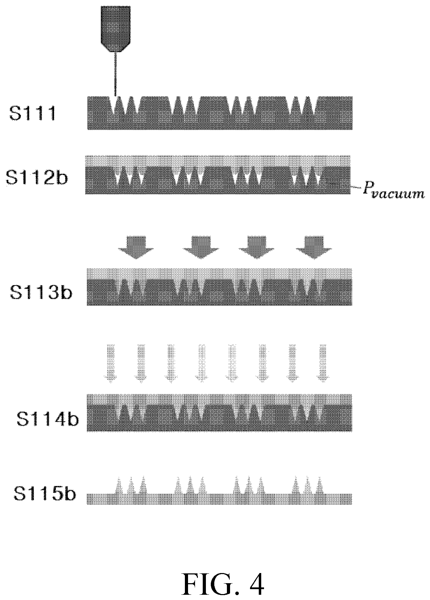

FIG. 4 is a flowchart illustrating a mold and imprint process S11 for forming an acrylic polymer layer, in the manufacturing process of a microneedle biosensor of FIG. 2. The process is configured by a mold manufacturing step S 111 of forming a groove having a shape corresponding to a microneedle on a polytetrafluoroethylene (PTFE) block with laser, a step S 112b of placing an acrylic UV resin on the mold in a vacuum state, a step S113b of pressurizing with a press after vacuum off, a UV curing step S114b, and a demolding step S115b. The acrylic microneedle has the advantage of having a short manufacturing process of approximately 5 to 10 minutes, and an acrylic microneedle has the advantage of good adhesiveness to Au.

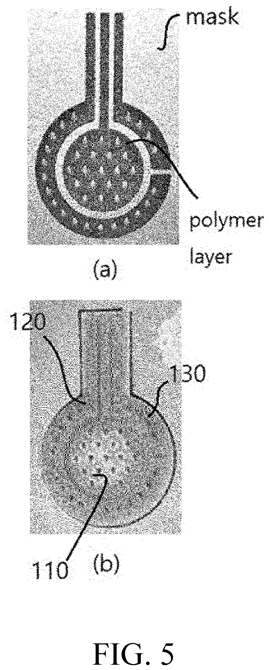

FIG. 5 is a view for explaining a metallization process in the manufacturing process of a microneedle biosensor of FIG. 2. According to the metallization process, shadow masks corresponding to the patterns of the working electrode 110, the counter electrode 120, and the reference electrode 130 of FIG. 1 are formed on the microneedle polymer layer manufactured by the imprint process of FIG. 3 or 4 and an Au or Au+Ti/Cr adhesive layer is sputtered to form a metal layer. In the patterns of the working electrode 110, the counter electrode 120, and the reference electrode 130, the working electrode 110 includes a first base which is a circular thin film, the counter electrode 120 includes a second base of a strip thin film type which forms a part of a second circumference spaced apart from the circumference of the first base with a setting distance to be concentric with the first base, and the reference electrode 130 includes a third base of a strip thin film type which is spaced apart from the other end of the second base with a setting interval and forms the second circumference with the strip shaped second base.

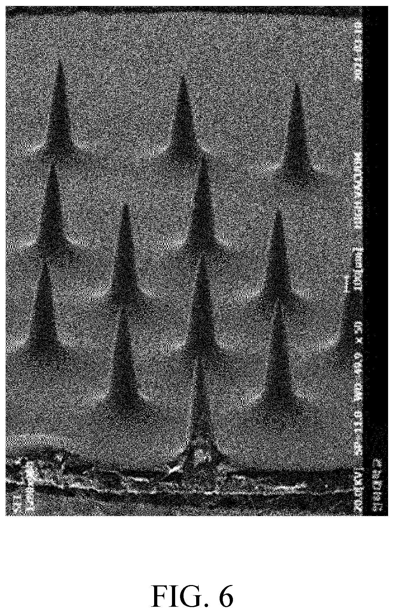

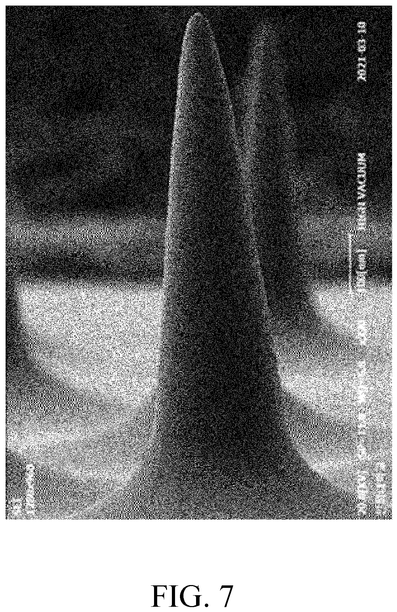

FIGS. 6 to 8 are views illustrating a state in which the passivation process of the manufacturing process of a microneedle biosensor of FIG. 2 is completed. In the passivation process, the UV adhesive is spin-coated on the Au surface and then dried to form the passivation layer. As the UV adhesive, NOA 68 is desirably applied.

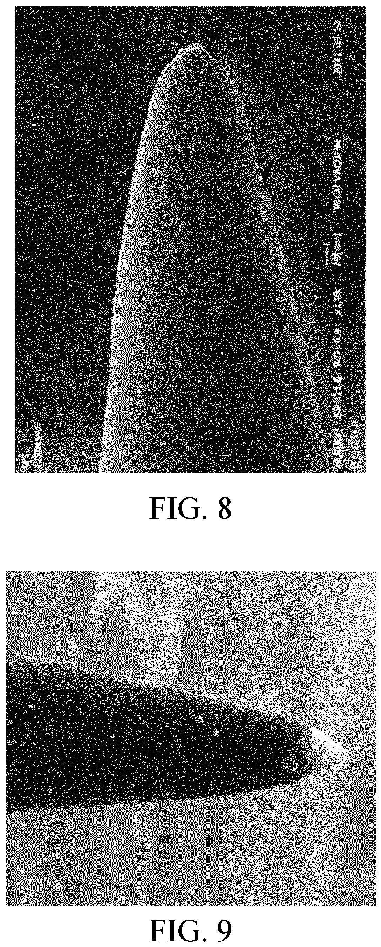

Hereinafter, the post-processing process S20 will be described with reference to FIGS. 9 to 12.

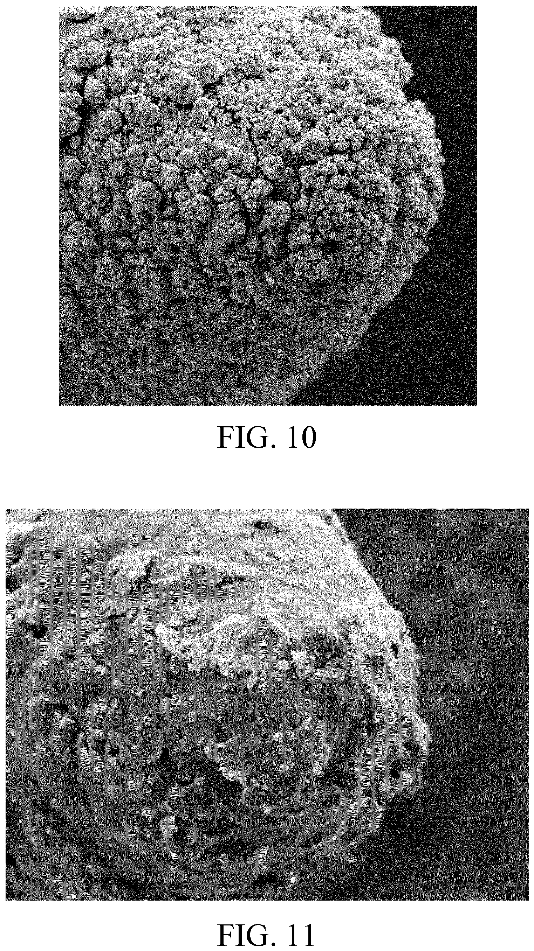

In a microneedle biosensor according to the exemplary embodiment of the present invention, it is desirable to coat the working electrode of the microneedle with Pt black as a glucose oxidation catalyst to measure a blood sugar. FIGS. 9 and 10 are views illustrating a tip of the needle plated with Pt-black. The plating is most desirably performed at a current of 5 mmA using 2.5 mmol chloroplatinic acid and 0.1 M HCl.

FIG. 11 is a view illustrating Pt-black further coated with nafion. Nafion is a biocompatible material to act as a shield to limit the access of various in-vivo signal interference materials to Pt black. Nafion is desirably coated using a spin coating method.

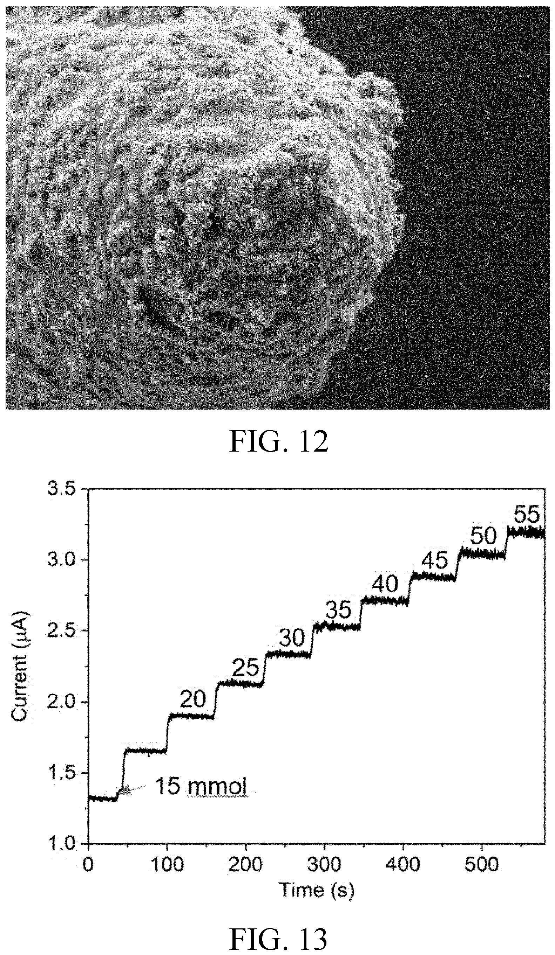

FIG. 12 is a view illustrating Ag/AgCl coated with nafion. The reference electrode may be formed by drop-casting the Ag/AgCl gel in the region of the reference electrode 130.

In the post-processing process S20, the region of the working electrode 110 is desirably coated with Pt-black and nafion and the reference electrode 130 is desirably coated with Ag/AgCl.

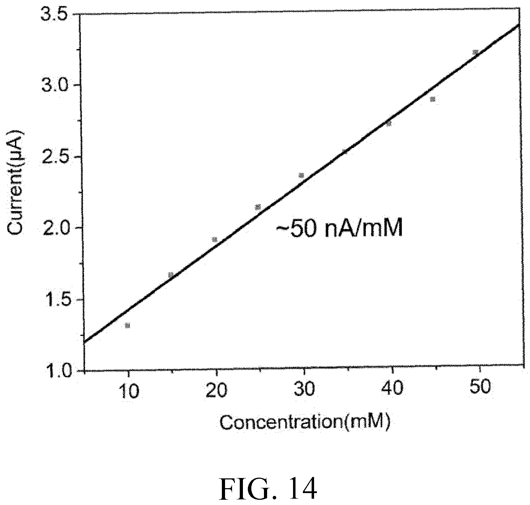

FIG. 13 is a graph obtained by measuring an output current when a glucose concentration is step-wisely increased from 15 mmol to 55 mmol and FIG. 14 is a graph illustrating a current which linearly increases in accordance with the glucose concentration. Accordingly, the accuracy of the measured glucose value using the microneedle biosensor according to the exemplary embodiment of the present invention may be known.

## Claims

1. A microneedle biosensor comprising:
a working electrode including a first base of a circular thin film type, a plurality of microneedles which perpendicularly protrudes on the first base, and a first wiring line which extends from one end of a circumference of the first base;
a counter electrode which includes a second base of a strip thin film type which forms a part of a second circumference spaced apart from the circumference of the first base with a setting distance to be concentric with the first base, a plurality of microneedles which perpendicularly protrudes on the second base, and a second wiring line which extends from one end of the second base to be horizontally disposed with the first wiring line; and
a reference electrode which includes a third base of a strip thin film type which is spaced apart from the other end of the second base with a setting interval and forms the second circumference with the strip shape of the second base, a plurality of microneedles which perpendicularly protrudes on the third base, and a third wiring line which extends from one end of the third base.

2. The microneedle biosensor of claim 1, wherein the second base occupies 3/4 of the second circumference and the third base occupies 1/4 of the second circumference.

3. The microneedle biosensor of claim 1, wherein the first wiring line perpendicularly extends from one end of the circumference of the first base, the second wiring line extends from one end of the second base to be horizontally disposed with the first wiring line, and the third wiring line extends from one end of the third base so as to be horizontally disposed with the first wiring line.

4. A manufacturing method of a microneedle biosensor, comprising:
a) forming a mold by forming grooves corresponding to shapes of microneedles of each of a working electrode, a counter electrode, and a reference electrode in a solid resin block;
b) imprinting a polymer layer of each of the working electrode, the counter electrode, and the reference electrode on the mold using polymer;
c) forming a metal layer by forming shadow masks corresponding to patterns of the working electrode, the counter electrode, and the reference electrode on the polymer layer and sputtering Au or Au+Ti/Cr adhesive layer; and
d) forming a passivation layer on the metal layer.

5. The manufacturing method of a microneedle biosensor of claim 4, further comprising:
after performing the step d),
plating with Pt-black and coating with nafion on tips of the microneedles of the working electrode.

6. The manufacturing method of a microneedle biosensor of claim 4, further comprising:
after performing the step d),
coating the microneedles of the reference electrode with Ag/AgCl.

7. The manufacturing method of a microneedle biosensor of claim 4, wherein the solid resin block is a polydimethylsiloxane (PDMS) or polytetrafluoroethylene (PTFE) material.

8. The manufacturing method of a microneedle biosensor of claim 4, wherein in the step b), the polymer is acryl or PLA.

9. The manufacturing method of a microneedle biosensor of claim 4, wherein in the step c), in the patterns of the working electrode, the counter electrode, and the reference electrode,
the working electrode includes a first base of a circular thin film type,
the counter electrode includes a second base of a strip thin film type which forms a part of a second circumference spaced apart from a circumference of the first base with a setting distance to be concentric with the first base, and the reference electrode includes a third base of a strip thin film type which is spaced apart from the other end of the second base with a setting interval and forms the second circumference together with the strip shape of the second base.
